(19) Europäisches Patentamt
European Patent Office
Office européen des brevets

(11) **EP 4 767 922 A1**

(12) **EUROPEAN PATENT APPLICATION**
published in accordance with Art. 153(4) EPC

(43) Date of publication:
**01.07.2026 Bulletin 2026/27**

(21) Application number: **24856863.6**

(22) Date of filing: **26.08.2024**

(51) International Patent Classification (IPC):
**A61B 3/00** (2006.01)　　**A61B 3/12** (2006.01)
**A61B 3/14** (2006.01)　　**G16H 50/20** (2018.01)
**G16H 50/50** (2018.01)　　**G16H 30/20** (2018.01)

(52) Cooperative Patent Classification (CPC):
**A61B 3/00; A61B 3/12; A61B 3/14; G16H 30/20;
G16H 50/20; G16H 50/50**

(86) International application number:
**PCT/KR2024/012729**

(87) International publication number:
**WO 2025/042252 (27.02.2025 Gazette 2025/09)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 24.08.2023 KR 20230111542
09.11.2023 KR 20230154293
09.11.2023 KR 20230154294
09.11.2023 KR 20230154295

(71) Applicant: **Mediwhale Inc.**
**Seoul 06049 (KR)**

(72) Inventors:
- **RIM, Hyung Taek**
  **Seoul 06601 (KR)**
- **TAN, Yong Yu**
  **Bukit Batok 650115 (SG)**

(74) Representative: **Westphal, Mussgnug & Partner
Patentanwälte mbB
Am Riettor 5
78048 Villingen-Schwenningen (DE)**

(54) **METHOD AND DEVICE FOR PREDICTING AND DIAGNOSING RISK LEVELS OF CARDIOVASCULAR DISEASES BY TYPE**

(57)　A device for predicting and diagnosing risk by cardiovascular disease type according to an embodiment comprises at least one processor, wherein the at least one processor predicts risk by cardiovascular disease type using a diagnostic model. The diagnostic model includes a first model that analyzes an inputted fundus image to predict cardiovascular risk; and a second model that receives a cardiovascular risk score predicted by the first model and disease type-specific factors to predict risk by cardiovascular disease type. Wherein the at least one processor is characterized by providing diagnostic information based on the cardiovascular risk predicted by the first model and the risk by cardiovascular disease type predicted by the second model.

EP 4 767 922 A1

FIG.9

**Description**

[Technical Field]

The present application relates to a diagnostic method and apparatus for predicting risk by cardiovascular disease type using a machine learning trained on fundus images and cardiovascular biomarkers.

[Background Art]

A fundus photograph (i.e., a fundus image or a retinal image) is an image capturing the posterior side of the retina and includes the retina, retinal blood vessels, optic nerve, macula, choroid, and the like. Fundus images have been traditionally used mainly for the diagnosis of ophthalmic diseases, but due to the rapid development of artificial intelligence (AI) technology, the scope of their application has significantly expanded to evaluating the risk of systemic diseases such as cardiovascular disease. This technology is recognized as a very useful tool, particularly in disease screening processes for large population groups, as it is non-invasive and cost-effective. AI exhibits excellent performance in analyzing retinal vascular changes associated with cardiovascular disease, thereby forming a new paradigm in the field of medical image analysis. As technologies for assisting the diagnosis of cardiovascular disease using existing fundus images, the applicant has previously proceeded with Korean patent applications such as Application Nos. 10-2018-0166720, 10-2018-0166721, and 10-2018-0166722. These patent applications relate to technologies for providing auxiliary information related to integrated risk assessment of cardiovascular disease, focusing on predicting the risk of cardiovascular disease more accurately through fundus images and supporting clinical decisions based thereon.

Meanwhile, cardiovascular disease includes detailed types such as myocardial infarction (MI), stroke, peripheral artery disease (PAD), heart failure (HF), atrial fibrillation (AF), and the like. While the applicant's existing patent applications mentioned above aim to predict the integrated risk for cardiovascular disease, if individual risks for each sub-type of cardiovascular disease can be predicted, more detailed auxiliary information can be provided. This can offer significant support for medical staff to make more precise clinical decisions tailored to each patient's condition.

[Detailed Description of the Invention]

[Technical Problem]

An object to be solved by the present invention is to provide a method for predicting risk by cardiovascular disease type for a target patient by receiving the patient's fundus image using a machine learning model trained on the correlation between fundus images and risks by type of cardiovascular disease, and a device implementing the same. Specifically, the present invention aims to provide a device that predicts risk by cardiovascular disease type by additionally considering disease type-specific factors including age, gender, blood biomarkers, smoking status, whether or not obese, and the like, based on an integrated cardiovascular risk score extracted from a fundus image by a machine learning model.

An object to be solved by the present invention is to provide a device that predicts risk by cardiovascular disease type by additionally considering a cardiovascular plaque type of a target patient, i.e., whether it is a soft plaque or a hard plaque.

An object to be solved by the present invention is to provide a cardiovascular disease type-specific risk prediction device including a model that determines which clinical tests are recommended for a patient based on the risk by cardiovascular disease type.

The problems to be solved by the present application are not limited to the aforementioned problems, and other problems not mentioned will be clearly understood by those of ordinary skill in the art from the present specification and the accompanying drawings.

[Technical Solution]

According to an aspect of the present application, a device for predicting and diagnosing risk by cardiovascular disease type according to an embodiment may include: a first model (cardiovascular risk prediction model) that analyzes an inputted fundus image to predict cardiovascular risk; and a second model (cardiovascular disease type-specific risk prediction model) that additionally receives a cardiovascular risk score predicted by the first model and disease type-specific factors to predict risk by cardiovascular disease type, characterized by providing a risk grade and related information for each cardiovascular disease type by synthesizing the cardiovascular risk predicted by the first model and the risk by cardiovascular disease type predicted by the second model.

According to an aspect of the present application, a device for predicting and diagnosing risk by cardiovascular disease type according to an embodiment may include: a first model that analyzes an inputted fundus image to predict cardiovascular risk; a second model that receives a cardiovascular risk score predicted by the first model and disease type-specific factors to predict risk by cardiovascular disease type; and a third model (recommended test determination model) that determines a clinical test recommended for a patient based on the risk by cardiovascular disease type predicted by the second model, characterized by providing cardiovascular diagnostic information including a risk grade and related information for each cardiovascular disease type, and recommended test-related information, by synthesizing the cardiovascular risk predicted by the first model and the risk by cardiovascular disease type predicted by the second model.

According to an aspect of the present application, a device for predicting and diagnosing risk by cardiovascular disease type according to an embodiment may include: a first model that analyzes an inputted fundus image to predict cardiovascular risk and a cardiovascular plaque type; a second model that additionally receives a cardiovascular risk score predicted by the first model and disease type-specific factors to predict risk by cardiovascular disease type; and a third model that determines a clinical test recommended for a patient based on the risk by cardiovascular disease type predicted by the second model, characterized by providing cardiovascular diagnostic information based on the cardiovascular risk and plaque type predicted by the first model and the risk by cardiovascular disease type predicted by the second model.

[Advantageous Effects]

According to the present invention, by using a machine learning model trained on the correlation between fundus images and risks by cardiovascular disease type, it is possible to predict not only an integrated cardiovascular risk score of a target patient but also risk by cardiovascular disease type by receiving the patient's fundus image. Additionally, the machine learning model can predict the patient's cardiovascular plaque type. Through this, it is possible to establish a customized treatment plan optimized for each patient and to identify patients at high risk of a specific cardiovascular disease early.

The effects of the present application are not limited to the aforementioned effects, and other effects not mentioned will be clearly understood by those of ordinary skill in the art from the present specification and the accompanying drawings.

[Brief Description of Drawings]

FIG. 1 is a block diagram for explaining a diagnostic device according to an embodiment.
FIGS. 2A and 2B are a diagram for explaining that cardiovascular risk (Reti-CVD) can be a biomarker for predicting individual risks of cardiovascular stroke, myocardial infarction, and atrial fibrillation.
FIGS. 3 and 4 are diagrams for explaining the correlation between Reti-CVD and various cardiovascular disease types.
FIGS. 5A, 5B and 6 are diagrams for explaining cardiovascular risk prediction performance for diabetic patients according to the present embodiment.
FIG. 7 is a diagram for explaining a method for predicting risk by cardiovascular disease type according to an embodiment.
FIGS. 8A and 8B are a diagram for explaining the prediction performance for atrial fibrillation risk according to an embodiment.
FIG. 9 is a diagram for explaining a method for predicting risk by cardiovascular disease type according to an embodiment.
FIG. 10 is a diagram for explaining a method for predicting risk by cardiovascular disease type according to another embodiment.

[Mode for Invention]

The above-mentioned objects, features, and advantages of the present application will become clearer through the following detailed description related to the accompanying drawings. However, since the present application can be modified in various ways and can have various embodiments, specific embodiments will be illustrated in the drawings and described in detail below.

Throughout the specification, the same reference numerals represent the same components in principle. In addition, components having the same function within the scope of the same idea appearing in the drawings of each embodiment will be described using the same reference numerals, and overlapping descriptions thereof will be omitted.

If a detailed description of a known function or configuration related to the present application is determined to unnecessarily obscure the subject matter of the present application, the detailed description thereof will be omitted. In addition, numbers used in the description process of the present specification (e.g., first, second, etc.) are merely identification symbols for distinguishing one component from other components.

In addition, the suffixes "module" and "part" for the components used in the following embodiments are given or used interchangeably only in consideration of the ease of writing the specification and do not have meanings or roles distinguished from each other by themselves.

In the following embodiments, singular expressions include plural expressions unless the context clearly indicates otherwise.

In the following embodiments, terms such as "comprise," "include," or "have" mean that features or components described in the specification exist and do not pre-exclude the possibility that one or more other features or components may be added.

In the drawings, components may be exaggerated or reduced in size for convenience of explanation. For example, the size and thickness of each component shown in the drawings are arbitrarily shown for convenience of explanation, and the present invention is not necessarily limited to the illustrated ones.

When an embodiment is differently implementable, the order of a specific process may be performed differently from the described order. For example, two processes described in succession may be performed substantially simultaneously or may be performed in an order opposite to the described order.

In the following embodiments, when components are said to be connected, it includes not only a case where the components are directly connected but also a case where the components are indirectly connected with other components interposed therebetween. For example, in the present specification, when components are said to be electrically connected, it includes not only a case where the components are directly electrically connected but also a case where the components are indirectly electrically connected with other components interposed therebetween.

Hereinafter, a diagnostic device and method for assisting the judgment of medical staff by predicting risk by cardiovascular disease type based on an ocular image will be described. In the present specification, the term "diagnosis" may mean "diagnostic assistance" for assisting the diagnosis of a disease rather than directly diagnosing the disease. Hereinafter, the term "diagnosis" is used for convenience of explanation, but this may mean "diagnostic assistance." The machine learning models described in the present specification may be designed based on various machine learning libraries.

Cardiovascular disease includes at least one of hypertension, atherosclerosis (or arterial sclerosis), coronary artery disease (CAD), valvular disease, pericardial disease, myocarditis, cardiomyopathy, arrhythmia, heart failure (HF), rheumatic heart disease, congenital heart disease, aortic aneurysm, peripheral vascular disease, cerebral vascular disease, or pulmonary vascular disease; and specifically, it may include at least one of angina pectoris, myocardial infarction (MI), pulmonary artery hypertension (PAH), cor pulmonale, left heart failure, right heart failure, aortic valve stenosis (AS), aortic valve regurgitation (AR), pulmonary valve stenosis (PS), pulmonary valve regurgitation (PR), tricuspid valve stenosis (TS), tricuspid valve regurgitation (TR), mitral valve stenosis (MS), mitral valve regurgitation, peripheral artery disease (PAD), aortic aneurysm (e.g., abdominal aortic aneurysm, thoracic aortic aneurysm), pericarditis, stroke, cerebral infarction, cerebral hemorrhage, cerebral aneurysm, thrombotic disease (e.g., deep vein thrombosis, pulmonary embolism), atrial fibrillation (AF), atrial tachycardia (AT), paroxysmal supraventricular tachycardia (PSVT), ventricular tachycardia (VT), bradyarrhythmia, ischemic stroke, hemorrhagic stroke, or transient ischemic attack (TIA). Cardiovascular disease may include complications. In addition, complications may include heart attack, death due to cardiovascular disease, cardiogenic shock, kidney disease, aspiration pneumonia, dysphagia, decreased motor function, decreased speech function, decreased cognitive function, sleep disorder, emotional disorder, neuropathic pain, urinary tract infection, malnutrition, deep vein thrombosis, pressure ulcers, falls, pain, seizures, or depression. Additionally, sub-types by cardiovascular disease type may be included. For example, heart failure is divided into heart failure with reduced ejection fraction (HFrEF), heart failure with mid-range ejection fraction (HFmrEF), or heart failure with preserved ejection fraction (HFpEF). In the case of stroke, it can be divided into ischemic stroke, thrombotic cerebral infarction (cerebral thrombosis), cerebral embolism (embolic cerebral infarction), lacunar infarction, hemorrhagic stroke, intracerebral hemorrhage, subarachnoid hemorrhage, transient ischemic attack, brainstem stroke, or stroke of unknown origin. In the case of atrial fibrillation, it includes sub-types of paroxysmal atrial fibrillation, persistent atrial fibrillation, long-standing persistent atrial fibrillation, or permanent atrial fibrillation; and in the case of myocardial infarction, it is divided into sub-types of type 1 (spontaneous myocardial infarction), type 2 (myocardial infarction secondary to an ischemic imbalance), type 3 (myocardial infarction resulting in death when biomarker values are unavailable), type 4a (myocardial infarction related to percutaneous coronary intervention (PCI)), type 4b (myocardial infarction related to stent thrombosis), or type 5 (myocardial infarction related to coronary artery bypass grafting (CABG)).

## 1. Biomarkers for Specific Cardiovascular Disease Sub-Types

A fundus image provides a method for non-invasively observing the vascular structure of the human body, through which a basis for predicting the possibility of occurrence of cardiovascular disease (CVD) is prepared. A machine learning model trained on fundus images and cardiovascular biomarkers is used to predict the risk of cardiovascular disease that may occur in the future. Principal cardiovascular disease biomarkers include coronary artery calcium (CAC) score, Pooled Cohort Equation (PCE) score, QRISK score, modified Framingham Risk Score (FRS), carotid intima-media thickness (CIMT) score, brachial-ankle pulse wave velocity (baPWV) score, or ankle-brachial index (ABI). The machine learning model generates Reti-CVD, which is a cardiovascular risk score for an inputted fundus image. According to this score, it is classified into three groups: low risk group, moderate risk group, and high risk group. For this classification criterion, reference values established in the applicant's previous study, "Deep-learning-based cardiovascular risk stratification using coronary artery calcium scores predicted from retinal photographs," The Lancet Digital Health 3(5), were used. The applicant revealed through previous research that the Reti-CVD score can be used as a biomarker for predicting the risk of cardiovascular disease that will occur in the future. In the present invention, it is newly revealed that the Reti-CVD score is closely related to the risks by various cardiovascular disease types that will occur in the future, and a detailed algorithm for predicting risks by cardiovascular disease type based on Reti-CVD is provided.

The diagnostic device can predict cardiovascular risk, risk by cardiovascular disease type, and risk by sub-type of the type, using machine learning models.

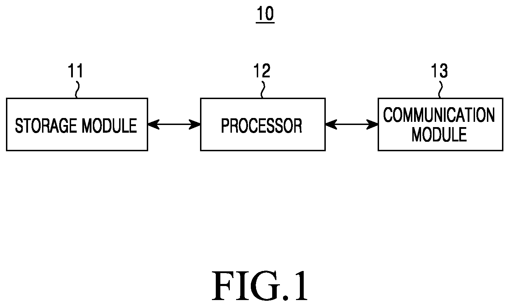

FIG. 1 is a block diagram for explaining a diagnostic device according to an embodiment. Referring to FIG. 1, the diagnostic device (10) may include a processor (12), a storage module (11), and a communication module (13).

The processor (12) may include at least one of a central processing unit (CPU), random access memory (RAM), graphic processing unit (GPU), one or more microprocessors, or other electronic components capable of processing inputted data according to a predetermined logic. In addition, there may be at least one processor (12).

The processor (12) can read a system program and various processing programs stored in the storage module (11). As an example, the processor (12) can develop a process, method, or the like for performing diagnosis, which will be described later, on the RAM and perform various treatments according to the developed program. For example, the processor (12) can process algorithms described in the present specification. The processor (12) can predict cardiovascular risk using a machine learning model. In addition, the processor (12) can implement various methods described in the present specification.

The storage module (11) can store a diagnostic model. The storage module (11) can store a machine learning model, parameters of the machine learning model, variables, algorithms described in the present specification, and the like.

The storage module (11) may be implemented as a non-volatile semiconductor memory, a hard disk, a flash memory, RAM, read-only memory (ROM), electrically erasable programmable read-only memory (EEPROM), or other tangible non-volatile recording media.

The storage module (11) can store various processing programs, parameters for performing processing of a program, or such processing result data. As an example, the storage module (11) can store a program and parameters for performing diagnosis described later and data obtained according to the performance of such a program. In addition, the storage module (11) can store various machine learning models described later.

Although not shown, the diagnostic device (20) may further include an input module. The input module can acquire a user input. For example, the input module can acquire a user input. In addition, the input module can acquire at least one of the subject's physical information (height, weight, age, gender, race, smoking status, blood pressure (e.g., blood pressure value, whether or not hypertension), whether or not diabetes (or blood glucose value), or cholesterol value) described in the present specification. In addition, the input module can acquire disease type-specific factors, which will be described later. The processor (12) can acquire pieces of information inputted through the input module.

The communication module (13) can communicate with various devices (e.g., a training device, a client device, etc.). As one example, the diagnostic device (10) may be implemented in the form of a server communicating with a client device. In addition, the diagnostic device (10) may be implemented in the form of a client device.

**<Example 1 - Prediction of Stroke, Myocardial Infarction, and Atrial Fibrillation Risk>**

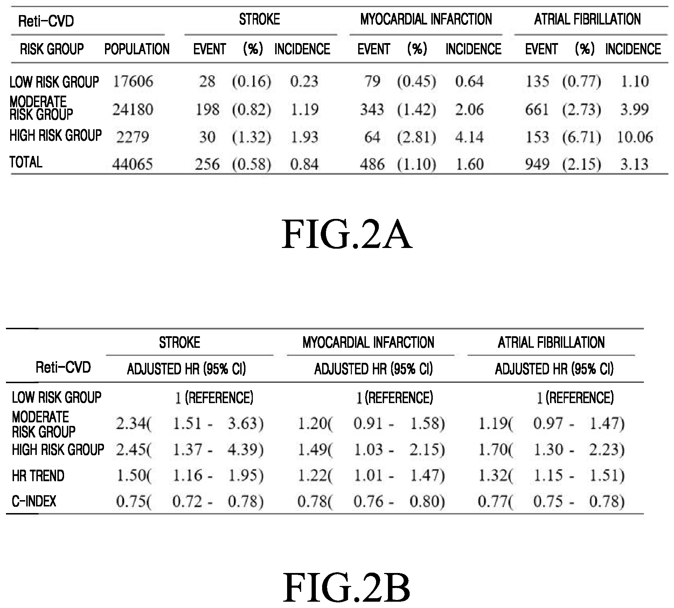

FIGS. 2A and 2B are a drawing for explaining that cardiovascular risk (Reti-CVD) can serve as a biomarker for predicting individual risks of cardiovascular stroke, myocardial infarction, and atrial fibrillation. FIG. 2A represents incidence rates of stroke, myocardial infarction, and atrial fibrillation in a low risk group, a moderate risk group, and a high risk group according to Reti-CVD scores. In addition, FIG. 2B shows an adjusted HR (95% CI), HR trend, and C-index in each risk group. Here, CI represents a confidence interval.

(1) Stroke: Reti-CVD scores showed a significant correlation with stroke occurrence risk. Analysis results showed that as the Reti-CVD score increased, the risk of stroke occurrence increased (adjusted HR trend, 1.50; 95% CI, 1.16-1.95; p=0.002). This suggests that Reti-CVD scores can be utilized as a useful biomarker for identifying high-risk patient groups requiring early preventive intervention.

(2) Myocardial Infarction (MI): Reti-CVD scores also showed a significant correlation with myocardial infarction (MI) occurrence risk. As the Reti-CVD score increased, the MI occurrence risk also increased (adjusted HR trend, 1.22; 95% CI, 1.01-1.47; p=0.036). This score can be utilized for early identification of individuals at high risk for myocardial infarction and for strengthening preventive measures.

(3) Atrial Fibrillation (AF): Reti-CVD scores were also closely associated with atrial fibrillation (AF) occurrence risk (adjusted HR trend, 1.32; 95% CI, 1.15-1.51; p<0.001). This suggests that Reti-CVD scores may be useful for assessing the risk of atrial fibrillation occurrence, and Reti-CVD can serve as an important tool for early risk assessment and establishment of preventive management strategies for atrial fibrillation.

**<Example 2 - Risk Prediction for 14 Cardiovascular Disease Types Including Myocardial Infarction, Peripheral Artery Disease, etc.>**

This example analyzes the correlations between Reti-CVD scores and 13 different cardiovascular diseases (CVD) as well as arterial hypertension. A cross-sectional analysis was performed on 45,980 participants from the UK Biobank, and logistic regression analysis was used to identify differential correlations with various cardiovascular diseases, with adjustments made for factors such as hypertension, diabetes, dyslipidemia, and smoking during the analysis process.

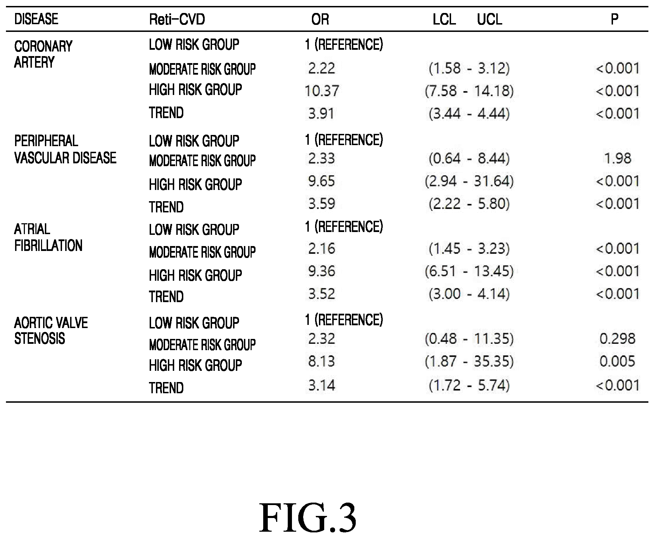

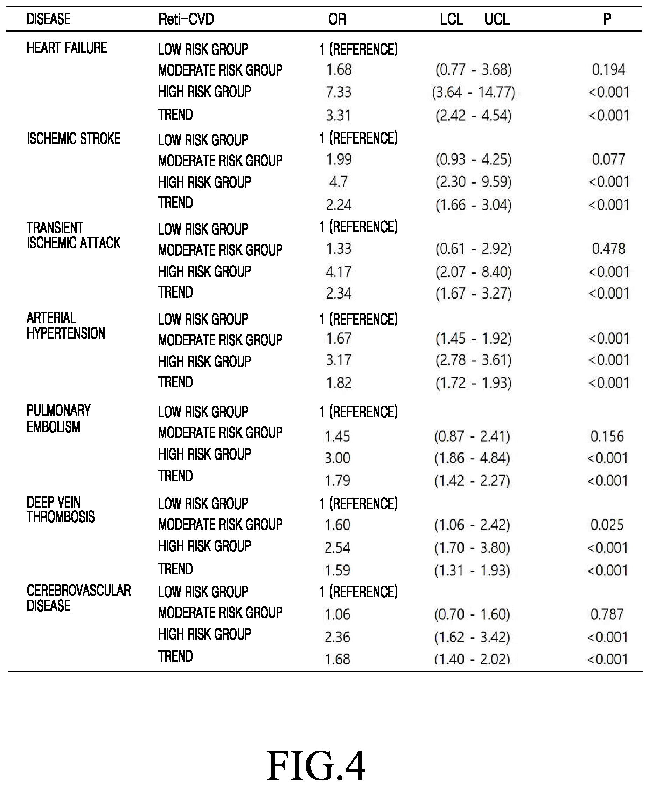

FIGS. 3 and 4 are drawings for explaining correlations between Reti-CVD and various cardiovascular disease types. Referring to FIGS. 3 and 4, (a) and (b) are tables for explaining the relationship between Reti-CVD scores and risk by cardiovascular disease type. In (a) and (b), OR represents an odds ratio, LCL represents a lower control limit, and UCL represents an upper control limit. In these tables, it was confirmed that the high risk group according to Reti-CVD was significantly associated with specific cardiovascular diseases compared to the low risk group.

(1) Coronary Artery Disease or Myocardial Infarction (MI): The risk of coronary artery disease occurrence increased significantly as the Reti-CVD score increased. For the high risk group, the odds ratio (OR) was 10.37 (95% CI, 7.58-14.18), and a very significant increase was observed in the overall trend.

(2) Peripheral vascular disease or peripheral artery disease (PAD): The risk of peripheral vascular disease occurrence also increased as the Reti-CVD score increased. The odds ratio for the high risk group was 9.65 (95% CI, 2.94-31.64), showing a statistically significant correlation.

(3) Atrial Fibrillation (AF): A strong correlation was also observed between Reti-CVD scores and the risk of atrial fibrillation occurrence, with an odds ratio of 9.36 (95% CI, 6.51-13.45) for the high risk group.

(4) Aortic Valve Stenosis (AVS): The risk of aortic valve stenosis occurrence increased as the Reti-CVD score increased, with an odds ratio of 8.13 (95% CI, 1.87-35.35) for the high risk group.

(5) Other diseases: Significant correlations with Reti-CVD scores were also confirmed in various cardiovascular diseases such as stroke, heart failure (HF), and pulmonary embolism (PE).

Examples 1 and 2 focused on analyzing correlations between Reti-CVD scores and various cardiovascular types and related conditions, and on predicting risk for each type. These results can be utilized to detect the potential existence of cardiovascular diseases unknown to the patient and to assess the risk thereof. That is, based on correlation data between Reti-CVD scores and various CVDs, the diagnostic device can detect cardiovascular diseases that may currently exist without the patient's awareness. This is useful for discovering diseases in conditions where the patient does not clearly feel symptoms or that have not been diagnosed at an early stage. Additionally, based on the study results, the diagnostic device can analyze Reti-CVD scores and existing patient health data to prioritize and recommend the cardiovascular disease that is currently most likely for the patient. This system can guide patients and medical staff to consider additional testing for potential risk diseases, and can promote early diagnosis and preventive treatment. For example, if a patient shows a high risk associated with atrial fibrillation (AF) according to the Reti-CVD score, there is a high possibility that the patient has undiagnosed atrial fibrillation, and the diagnostic device can recommend an additional electrocardiogram test or monitoring. For another patient showing a moderate risk related to peripheral vascular disease (PVD) or peripheral artery disease (PAD), the diagnostic device can guide the patient to receive tests more intensively for this disease with a high possibility of occurrence.

**2. Cardiovascular Prediction Biomarkers for Patients with Existing Diseases**

Reti-CVD can be a cardiovascular risk index that can more precisely predict the risk of secondary cardiovascular-related diseases by considering the patient's gender, race, and existing disease state. This score can be derived through a model trained on fundus images and coronary artery calcium (CAC) data. Reti-CVD scores basically assess the risk of cardiovascular disease by analyzing a subject's fundus image, but can evolve into a prediction tool that comprehensively considers the patient's existing disease state by expanding this. Through this, it is possible to more accurately predict the risk of secondary cardiovascular diseases in high-risk groups, for example, patients with chronic diseases such as diabetes, hypertension, and hyperlipidemia, or patients who have already experienced cardiovascular diseases (heart failure, coronary artery disease, stroke, etc.). For instance, the onset patterns of cardiovascular disease may differ according to gender (male/female) and race. By additionally reflecting such demographic variables in Reti-CVD scores, cardiovascular risk in a specific gender or race can be individually predicted. In addition, patients with chronic diseases such as hypertension, hyperlipidemia, and diabetes may have an increased risk of additional cardiovascular diseases. The Reti-CVD-based risk prediction model according to the present invention can predict the risk of a patient secondarily contracting cardiovascular diseases (e.g., heart failure, coronary artery disease, stroke, etc.) by considering such existing diseases. Patients already suffering from cardiovascular diseases (heart failure, coronary artery disease, stroke, etc.) have additional risk for the occurrence of new cardiovascular disease types. Reti-CVD scores can be useful for more accurately assessing the risk of secondary cardiovascular disease occurrence in such patient groups.

**<Example 1 - Prediabetic and Diabetic Patients>**

In this example, prediabetic and diabetic patient data from the UK Biobank were used to classify patients into three groups: a low risk group, a moderate risk group, and a high risk group based on Reti-CVD scores, and fatal and non-fatal cardiovascular disease types (coronary artery disease, ischemic stroke, transient ischemic attack) were evaluated while tracking and observing the patients.

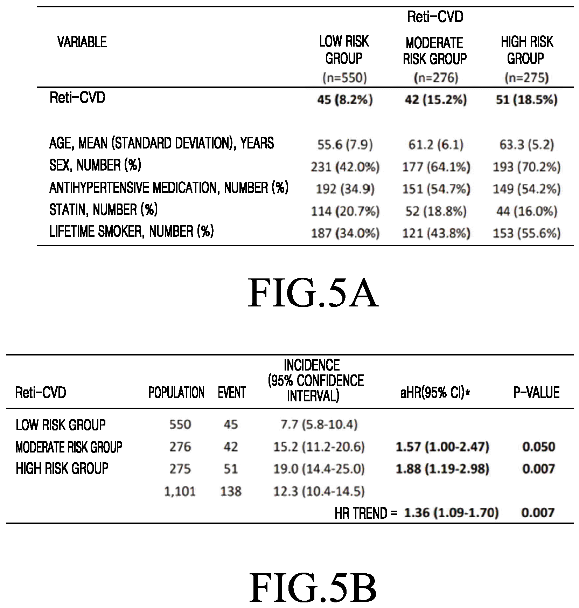

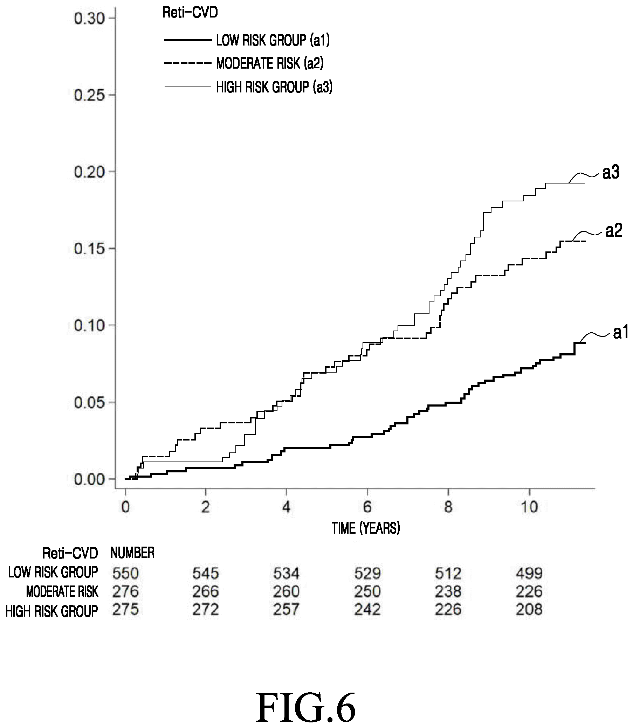

FIGS. 5A, 5B and 6 are drawings for explaining cardiovascular disease prediction performance for diabetic patients according to the present embodiment. These drawings show results classified into a low risk group (n=550), a moderate risk group (n=276), and a high risk group (n=275) in a 2:1:1 ratio according to the 50th and 75th percentiles based on Reti-CVD scores. This cardiovascular disease prediction score was derived based on retinal images of prediabetic and diabetic patients. To evaluate how well the Reti-CVD score predicts fatal and non-fatal cardiovascular events, a survival analysis using a Cox proportional hazards model and hazard ratios (HRs) was performed using longitudinal data from the UK Biobank. As shown in FIGS. 5A, 5B and 6, among 1,101 prediabetic and diabetic patients, 138 (12.5%) experienced cardiovascular disease events. During a median follow-up period of 11 years, such events occurred in 8.2% (45/550) of the low risk group, 15.2% (42/276) of the moderate risk group, and 18.5% (51/275) of the high risk group. Even after adjusting for factors such as age, gender, antihypertensive medication use, statin use, and lifetime smoker records, a significant association was observed between the cardiovascular disease prediction score and the cardiovascular event incidence rate. In particular, compared to the low risk group, the risk of cardiovascular event occurrence significantly increased in the moderate risk group (hazard ratio 1.57, 95% CI, 1.00-2.47) and the high risk group (hazard ratio 1.88, 95% CI, 1.19-2.98). In addition, a trend of the hazard ratio gradually increasing (HR trend 1.36, 95% CI, 1.09-1.70) was observed. This result suggests that the Reti-CVD score can be utilized as a useful tool for risk stratification among prediabetic and diabetic patients and shows that it has important potential in managing high-risk patients.

**3. Method for Predicting Risk by Cardiovascular Disease Type**

**3.1 Direct Prediction Model (Classification Model) for Risk by Cardiovascular Disease Type**

The diagnostic device may perform the following operations to directly predict risk by cardiovascular disease type. A machine learning (deep learning) model performed in the diagnostic device can be trained on inputted fundus images and the presence or absence of cardiovascular disease types such as atrial fibrillation (AF), coronary artery disease (CAD), peripheral artery disease (PAD), heart failure (HF), and stroke as labels. Through this learning process, features associated with cardiovascular diseases are learned and recognized. The processor of the diagnostic device can predict the possibility of occurrence of cardiovascular disease types for the subject of the fundus image based on the analysis results. Deep learning algorithms for predicting risk by multiple cardiovascular disease types from fundus images may be designed in various ways. These may include a single model, a parallel model, and a multi-task model, among others. A single model approach is a method of simultaneously predicting multiple cardiovascular disease types (e.g., atrial fibrillation (AF), coronary artery disease (CAD), peripheral artery disease (PAD), heart failure (HF), stroke) using a single deep learning model. This model receives a fundus image as input and has output nodes that individually predict the likelihood of occurrence for various cardiovascular disease types. The final layer of the model has multiple output nodes, and each node returns the likelihood of occurrence of a specific cardiovascular disease type as a probability. In the parallel model approach, individual deep learning models are constructed for each cardiovascular disease type. For example, a model for predicting atrial fibrillation (AF), a model for predicting coronary artery disease (CAD), a model for predicting peripheral artery disease (PAD), etc., each exist independently. Each model receives a fundus image as input and predicts only the cardiovascular disease type it targets. A multi-task model approach is similar to a single model, but is a method that strengthens the learning of common features for predicting multiple cardiovascular disease types. A single model learns common features, and based on these, performs individual predictions for each cardiovascular disease type. This model is designed in a manner that learns features related to multiple cardiovascular disease types in common layers, and then branches for each cardiovascular disease type. Each branched path performs detailed predictions tailored to the corresponding cardiovascular disease type.

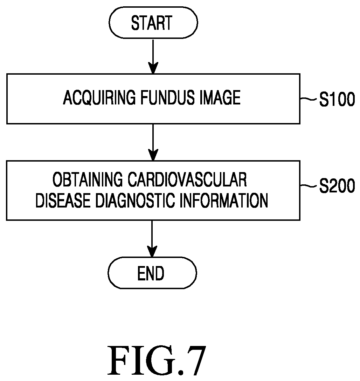

FIG. 7 is a drawing for explaining a method for predicting risk by cardiovascular disease type according to one embodiment.

Referring to FIG. 7, the processor of the diagnostic device may include a step of acquiring a fundus image (S100) and a step of obtaining cardiovascular disease diagnostic information (S200).

In step S100, the processor of the diagnostic device may acquire a fundus image. Additionally, depending on the embodiment, the processor of the diagnostic device may perform preprocessing, augmentation, serialization, and the like on the obtained fundus image. Since various techniques may be applied thereto, a detailed description will be omitted.

Additionally, in step S200, the processor of the diagnostic device may obtain cardiovascular disease diagnostic information. In this specification, diagnostic information may be expressed as diagnostic assistance information. Cardiovascular disease diagnostic information may include risk prediction information by cardiovascular disease type. In this specification, risk prediction information by cardiovascular disease type may include risk prediction information for cardiovascular disease types such as stroke, myocardial infarction (MI), atrial fibrillation (AF), heart failure (HF), and peripheral artery disease (PAD). For example, the risk prediction information by cardiovascular disease type may include information on a probability value (score) and/or a grade for the occurrence of the corresponding cardiovascular disease type in the subject of the fundus image. Additionally, the risk prediction information by cardiovascular disease type may include information on a probability value (score) and/or a grade for the occurrence of the corresponding cardiovascular disease type within a predetermined period (e.g., within 10 years, within 5 years) in the subject of the fundus image. Additionally, secondary auxiliary information described below may be obtained in step S200.

Hereinafter, specific implementation examples will be described.

### <Example 1 - Reti-AF Single Model>

This embodiment shows the conceptual operation of the Reti-AF algorithm for predicting atrial fibrillation (AF) risk. The Reti-AF algorithm uses a patient's fundus image (retinal image) as input and recognizes patterns related to the presence or absence of atrial fibrillation (AF) by learning features of the retinal image. Through the analysis results, the algorithm predicts whether the inputted image is associated with atrial fibrillation (AF) or is in a non-AF state. The Reti-AF algorithm may be performed by the processor of the diagnostic device.

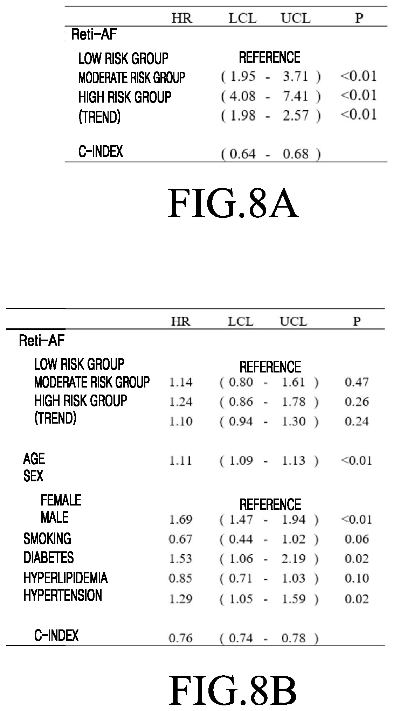

To evaluate the performance of the deep learning algorithm Reti-AF according to this embodiment, univariable Cox regression and multivariable Cox regression were performed. FIGS. 8A and 8B are a drawing for explaining the prediction performance for atrial fibrillation risk according to one embodiment. Referring to FIGS. 8A and 8B, FIG. 8A is a table where contents related to atrial fibrillation risk (Reti-AF) are summarized through univariable Cox regression, and FIG. 8B is a table where contents related to atrial fibrillation risk (Reti-AF) are summarized through multivariable Cox regression.

As a result of univariable Cox regression, it was shown that AF incidence significantly increased when the Reti-AF score was high (in the unadjusted state for variables such as age and gender). The hazard ratio (HR) for the high risk group was 5.50 (95% CI, 4.08-7.41), demonstrating that Reti-AF is effective in predicting AF risk. In multivariable Cox regression, Reti-AF maintained a significant association with AF occurrence even after adjusting for variables such as age, gender, smoking status, diabetes, hyperlipidemia, and hypertension. In particular, age acted as an independently significant variable for AF occurrence and strengthened the predictive performance of Reti-AF. The C-index of the multivariable model was 0.76, showing improved performance compared to univariable analysis. In conclusion, the Reti-AF algorithm demonstrated significant performance in predicting the risk of AF occurrence.

### 3.2 Risk Prediction Model by Cardiovascular Disease Type Based on Cardiovascular Biomarker (Reti-CVD)

#### 3.2.1 Basic Algorithm Configuration

Patients already suffering from cardiovascular diseases (heart failure, coronary artery disease, stroke, etc.) have additional risk for the occurrence of new specific cardiovascular sub-diseases. Accordingly, it is necessary to predict the risk of occurrence of each specific cardiovascular sub-disease.

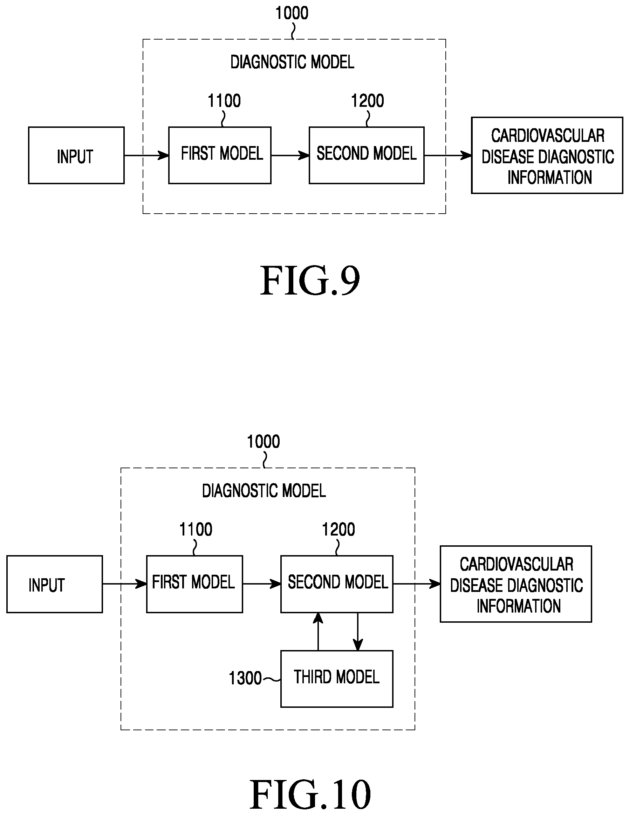

FIG. 9 is a drawing for explaining a method for predicting risk by cardiovascular disease type according to one embodiment. FIG. 9 shows the process of assessing the overall cardiovascular risk of a patient through the Reti-CVD prediction model (diagnostic model (1000)) (first model (1100)), and predicting risk by cardiovascular disease type through analysis including disease type-specific factors (second model (1200)). The first model (1100) and the second model (1200) may be included in the diagnostic model (1000).

**Cardiovascular risk prediction model (First Model'):** In this step, the patient's overall cardiovascular risk is assessed through the first model (1100). The first model (1100) may learn the patient's fundus image and coronary artery calcium (CAC) score to predict Reti-CVD, which is a cardiovascular risk score, according to the inputted fundus image. Additionally, the first model (1100) may predict various disease type-specific factors in addition to the Reti-CVD score. For example, disease type-specific factors may include at least one of parameters representing the subject's physical information, such as biological age, gender, height, weight, BMI index, information about obesity (obesity value, BMI, body mass, body fat percentage, body muscle mass, whether or not obese, etc.).

Disease type-specific factors (laboratory markers) may include at least one of diagnostic value parameters such as hematocrit level, red blood cell count, white blood cell count, hemoglobin level, platelet count, total iron-binding capacity (TIBC), iron level, ferritin (storage iron protein) level, total protein level, albumin level, aspartate transaminase (AST) level, alanine transaminase (ALT) level, $\gamma$-GT, alkaline phosphatase (ALP) level, globulin level, hepatitis antigen level, hepatitis antibody level, blood glucose level, glycated hemoglobin (HbA1C) level, blood urea nitrogen (BUN) level, creatinine (Cr) level, uric acid level, total cholesterol level, high-density lipoprotein (HDL cholesterol) level, low-density lipoprotein (LDL cholesterol) level, triglyceride (TG) level, apolipoprotein B (ApoB), lipoprotein(a) (Lp(a)), C-reactive protein (CRP), erythrocyte sedimentation rate (ESR), bicarbonate level, pH level, hematuria, urine occult blood level, urine protein level, urine glucose level, urine pH, urine bilirubin level, urine ketone body level, urine urobilinogen level, urine leukocyte count, urine creatinine level, urine albumin level, or urine albumin/creatinine ratio. Additionally, disease type-specific factors may include at least one of an electrocardiogram (ECG), systolic blood pressure (SBP), diastolic blood pressure (DBP), family history information, or patient clinical history information.

Meanwhile, the first model (1100) may learn the correlation between the fundus image and cardiovascular plaque types, i.e., the presence or absence of soft plaques and hard plaques, to predict a cardiovascular plaque type according to the inputted fundus image. Contents related to cardiovascular plaque type prediction are described in detail in the section 3.2.4 Algorithm Reflecting Cardiovascular Plaque Types.

**Prediction model for risk by cardiovascular disease type (Second Model'):** In this step, risk by cardiovascular disease type (e.g., atrial fibrillation, coronary artery disease, stroke, etc.) is predicted by utilizing data derived from the cardiovascular risk prediction model. Risk for each disease type is evaluated by synthesizing the Reti-CVD score obtained from the first model (1100) and the disease type-specific factors. Data inputted to the second model (1200) may be a feature vector of the fundus image extracted by the first model (1100) instead of the Reti-CVD score. Meanwhile, the disease type-specific factors can be predicted by the first model as mentioned above, in which case the prediction result (output result) of the factors by type can be inputted to the second model (1200).

The second model (1200) may further include an input module. The input module can acquire the disease type-specific factors through a user input. As shown in FIG. 9, if separate patient data (e.g., gender, age, blood test results, smoking status, whether or not obese, etc.) exist, the corresponding patient data can be inputted to the prediction model for risk by cardiovascular disease type. This model individually analyzes the likelihood of occurrence for each cardiovascular sub-disease to predict which cardiovascular disease the patient is more vulnerable to.

**Output of cardiovascular disease diagnostic Information:** In the final step, cardiovascular disease diagnostic information can be acquired and outputted by synthesizing the overall cardiovascular risk (integrated score) and the risk for each type. The corresponding diagnostic information may include the current patient state, risk for each type, and related explanatory information, and additionally, auxiliary information for establishing personalized prevention and treatment plans (e.g., secondary auxiliary information to be described later) can be provided.

<Example - Specific Patient Cases>

The risk prediction model by cardiovascular disease type independently evaluates the risk for each type for each patient by considering the Reti-CVD score and type-specific covariates.

[Table 1]

| 1. Case of Patient A | |
|---|---|
| Comprehensive State | High Reti-CVD score, hypertension, smoking history, female, age 65 |
| Stroke Risk | **Prediction:** Very high.<br>**Explanation:** Patient A is predicted to have a very high risk of stroke due to the overlap of the high Reti-CVD score, hypertension, smoking history, and the factors of being 65 years old and female. Since all these factors significantly contribute to an increase in risk in the stroke model, the patient has a very high possibility of stroke occurrence. |

(continued)

| 1. Case of Patient A | |
|---|---|
| Myocardial Infarction (MI) Risk | **Prediction:** High.<br>**Explanation:** Patient A has a high risk of myocardial infarction due to the high Reti-CVD, hypertension, smoking history, and age (65). However, menopausal status and LDL cholesterol levels have a major influence; if the patient is postmenopausal or the LDL cholesterol level is high, the risk of myocardial infarction can increase further. |
| Atrial Fibrillation (AF) Risk | **Prediction:** Moderate.<br>**Explanation:** For Patient A, the risk of atrial fibrillation is predicted to be at a moderate level due to the high Reti-CVD, hypertension, smoking history, and age (65). While obesity is a major factor, the risk may remain at a moderate level if obesity is absent. |
| Heart Failure (HF) Risk | **Prediction:** High.<br>**Explanation:** For Patient A, age (65), hypertension, and smoking history are major risk factors for heart failure. Since the Reti-CVD score is high and multiple risk factors overlap, the heart failure risk is predicted to be high. |
| Peripheral Artery Disease (PAD) Risk | **Prediction:** High.<br>**Explanation:** For Patient A, smoking history, hypertension, age (65), and being female all act as factors that increase the risk of peripheral artery disease. Along with the high Reti-CVD score, the risk of PAD is predicted to be high. |

[Table 2]

| 2. Case of Patient B | |
|---|---|
| Comprehensive State | Moderate Reti-CVD score, low cholesterol level, obesity, family history of heart disease, male, age 50. |
| Stroke Risk | **Prediction:** Moderate.<br>**Explanation:** Patient B has a moderate Reti-CVD score and obesity, but does not have hypertension or a smoking history, which are major risk factors for stroke. Considering that the patient is male and age 50, the stroke risk is predicted to be at a moderate level. |
| Myocardial Infarction (MI) Risk | **Prediction:** High.<br>**Explanation:** Due to the factors of obesity, family history of heart disease, and being male, Patient B has a high risk of myocardial infarction. Although the Reti-CVD score is moderate and the LDL cholesterol level is low, obesity and family history act as important prediction factors for myocardial infarction, resulting in a high risk prediction. |
| Atrial Fibrillation (AF) Risk | **Prediction:** High.<br>**Explanation:** Patient B has a high risk of atrial fibrillation due to obesity and the age of 50. Despite the moderate Reti-CVD score, the risk of atrial fibrillation is predicted to be high due to being male, obesity, and the age. |
| Heart Failure (HF) Risk | **Prediction:** Moderate.<br>**Explanation:** Although Patient B has obesity and a family history of heart disease, considering the relatively young age of 50 and the moderate Reti-CVD score, the heart failure risk is predicted to be at a moderate level. |
| Peripheral Artery Disease (PAD) Risk | **Prediction:** Moderate.<br>**Explanation:** For Patient B who is obese, the PAD risk may be somewhat high, but considering the moderate Reti-CVD score and age (50), the risk is predicted to be at a moderate level. The low LDL cholesterol is also a factor that reduces risk |

3.2.2 Detailed Algorithms of the Risk Prediction Model by Cardiovascular Disease Type (Second Model)

For each cardiovascular disease type (stroke, myocardial infarction (MI), atrial fibrillation (AF), heart failure (HF), or peripheral artery disease (PAD)), the model can consider the Reti-CVD score and type-specific covariates. Specific covariates by type include, for example, blood pressure, transient ischemic attack (TIA) history, atrial fibrillation state, and smoking state for stroke; LDL cholesterol, family history of heart disease, smoking, and hypertension for myocardial infarction (MI); and age, history of heart disease, obesity, and hypertension for atrial fibrillation (AF). In addition, for heart failure (HF), they may include age, history of heart disease, and smoking; and for peripheral artery disease (PAD), they may include smoking, diabetes, hypertension, hyperlipidemia, and age.

The processor of the diagnostic device can use a multivariate Cox proportional hazards model or other appropriate statistical or machine learning models for each specific disease type. The following models may be included in the second model (1200). The following are examples of risk formulas for each disease type performed by the respective models.

**(1) Stroke Model:**

$$\text{Stroke Risk} = \exp(\beta 1 \times \text{Reti-CVD} + \beta 2 \times \text{Blood Pressure} + \beta 3 \times \text{TIA History} + \beta 4 \times \text{AF Status} + \beta 5 \times \text{Smoking} + ...)$$

**(2) Myocardial Infarction (MI) Model:**

Myocardial Infarction Risk = $\exp(\beta 1 \times \text{Reti-CVD} + \beta 2 \times \text{LDL Cholesterol} + \beta 3 \times \text{Family History of Heart Disease} + \beta 4 \times \text{Smoking} + \beta 5 \times \text{Hypertension} + ...)$

**(3) Atrial Fibrillation (AF) Model:**

Atrial Fibrillation Risk = $\exp(\beta 1 \times \text{Reti-CVD} + \beta 2 \times \text{Age} + \beta 3 \times \text{History of Heart Disease} + \beta 4 \times \text{Obesity} + \beta 5 \times \text{Hypertension} + ...)$

**(4) Heart Failure (HF) Model:**

Heart Failure Risk = $\exp(\beta 1 \times \text{Reti-CVD} + \beta 2 \times \text{Age} + \beta 3 \times \text{Smoking} + \beta 4 \times \text{History of Heart Disease} + ...)$

**(5) Peripheral Artery Disease (PAD) Model:**

Peripheral Artery Disease Risk = $\exp(\beta 1 \times \text{Reti-CVD} + \beta 2 \times \text{Smoking} + \beta 3 \times \text{Diabetes} + \beta 4 \times \text{Hypertension} + \beta 5 \times \text{Hyperlipidemia} + \beta 6 \times \text{Age} + ...)$

In the above formulas, $\beta 1$, $\beta 2$, etc., represent coefficients for each covariate, and each coefficient reflects the influence of the covariate on the risk. Each model outputs a risk estimate (e.g., hazard ratio or probability) for a specific disease type (stroke, myocardial infarction (MI), atrial fibrillation (AF), heart failure (HF), or peripheral artery disease (PAD)). These risk estimates are calculated independently of each other, and although each model uses the Reti-CVD score as a common input, the result values can be individually produced. In addition, since these risk estimates represent individual probabilities or hazard ratios for different outcomes, they are not summed. Each risk can be independently interpreted based on the combination of the Reti-CVD score and the disease type-specific factors. Each model can operate independently. That is, the risk for each cardiovascular type (stroke, myocardial infarction, atrial fibrillation, heart failure, or peripheral artery disease) is calculated based on the Reti-CVD score and the covariates by type, and these models do not affect each other, and risk prediction for each type can be independently performed.

**3.2.3 Algorithm Including the Recommended Test Determination Model**

FIG. 10 is a drawing for explaining a method for predicting risk by cardiovascular disease type according to another embodiment.

Referring to FIG. 10, a recommended test determination model may be added to the basic algorithm configuration introduced in 3.2.1. The descriptions given above can be applied to the first model (cardiovascular risk prediction model, 1100) and the second model (risk prediction model by cardiovascular disease type, 1200), so detailed descriptions thereof will be omitted. The third model (1300) may also be included in the diagnostic model (1000).

**Recommended test determination model (Third Model'):** The third model (1300) determines what additional clinical test is recommended for the patient based on the risk by cardiovascular disease type, and the processor of the diagnostic device can provide information on the additional clinical test. Here, the additional clinical test may include an electrocardiogram (ECG), an echocardiogram, an ABI (Ankle-Brachial Index), an ABPI (Ankle-Brachial Pressure index), a blood test, a urinalysis, or an ultrasound.

For example, if stroke risk is predicted to be high, additional blood pressure monitoring or brain imaging tests may be recommended. If myocardial infarction risk is high, additional blood tests (e.g., checking LDL level) or heart function tests may be necessary. If heart failure risk is high, it is necessary to evaluate heart function in more detail through an echocardiogram or a BNP (B-type natriuretic peptide) test. If peripheral artery disease (PAD) risk is high, evaluation of blood flow status through a lower extremity Doppler ultrasound test, an ankle-brachial index (ABI), or other vascular tests may be recommended. In addition, if AF risk is predicted to be high, ECG monitoring (e.g., 24-hour Holter monitoring) may be recommended to detect paroxysmal atrial fibrillation (paroxysmal AF). Even in the cases of heart failure, peripheral artery disease, and atrial fibrillation, patients may also be advised to perform measurements through related mobile service apps, such as a heart rate monitoring app or a pain record app.

<Example - Output Example of Recommended Tests for Patients A and B>

This embodiment shows the process of determining whether an additional clinical test is necessary based on the risk prediction by cardiovascular disease type for Patients A and B. For disease types whose risk is predicted to be high according to each patient's condition, appropriate additional tests are recommended, through which the patient's cardiovascular health can be precisely evaluated and managed.

[Table 3]

| 1. Case of Patient A | |
|---|---|
| Comprehensive State | High Reti-CVD score, hypertension, smoking history, female, age 65 |
| Stroke Risk | **Prediction:** Very high.<br>**Recommended Test:** Since Patient A's stroke risk is predicted to be very high, an additional clinical test is necessary. Specifically, **blood pressure monitoring** and **brain Imaging tests** (e.g., MRI or CT) are recommended. Such tests will help identify the possibility of stroke occurrence early and establish preventive treatment plans. |
| Myocardial Infarction (MI) Risk | **Prediction:** High.<br>**Recommended Test:** Since Patient A's myocardial infarction risk is predicted to be high, an additional blood test (e.g., **LDL cholesterol level confirmation)** and **a heart function test** (e.g., echocardiogram) are recommended. Such tests help more accurately evaluate risk factors for myocardial infarction and modify treatment plans if necessary. |
| Atrial Fibrillation (AF) Risk | **Prediction:** Moderate.<br>**Recommended Test:** Although Patient A's atrial fibrillation risk is predicted to be moderate, considering the age (65), **ECG monitoring** (e.g., 24-hour Holter monitoring) may be considered. This can be useful for early detection of paroxysmal atrial fibrillation (paroxysmal AF). |
| Heart Failure (HF) Risk | **Prediction:** High.<br>**Recommended Test:** Since Patient A's heart failure risk is predicted to be high, it is necessary to evaluate heart function in more detail through **an echocardiogram** and **a BNP (B-type natriuretic peptide) test.** Such tests will help monitor the progression of heart failure and plan appropriate treatment. |

(continued)

| 1. Case of Patient A | |
| --- | --- |
| Peripheral Artery Disease (PAD) Risk | **Prediction:** High.<br>**Recommended Test:** Since Patient A's peripheral artery disease risk is predicted to be high, evaluation of blood flow status through **a lower extremity Doppler ultrasound test, an ankle-brachial Index test,** or **other vascular tests** is recommended. This enables confirmation of whether peripheral vascular stenosis exists and allows for immediate treatment if necessary. |

[Table 4]

| 2. Case of Patient B | |
| --- | --- |
| Comprehensive State | Moderate Reti-CVD score, low cholesterol level, obesity, family history of heart disease, male, 50 years old. |
| Stroke Risk | **Prediction:** Moderate.<br>**Recommended Test:** Since Patient B's stroke risk is predicted to be moderate, an additional test is not essential. **Regular blood pressure monitoring** and **lifestyle Improvement** are recommended. Continuous observation of the state is possible through periodic retinal imaging. |
| Myocardial Infarction (MI) Risk | **Prediction:** High.<br>**Recommended Test:** Since Patient B's myocardial infarction risk is predicted to be high, **non-invasive heart tests** (e.g., exercise/pharmacological stress testing) and **an echocardlogram** should be considered. These will help in early detection and prevention of heart disease. |
| Atrial Fibrillation (AF) Risk | **Prediction:** High.<br>**Recommended Test:** Since Patient B's atrial fibrillation risk is predicted to be high, **ECG monitoring** (e.g., 24-hour Holter monitoring) is recommended. This can help detect early signs of atrial fibrillation and correct cardiac rhythm early. |
| Heart Failure (HF) Risk | **Prediction:** Moderate.<br>**Recommended Test:** Since Patient B's heart failure risk is predicted to be moderate, it is good to monitor heart function through periodic **echocardiogram tests** and **BNP tests.** Additional tests can be determined according to the current state. |
| Peripheral Artery Disease (PAD) Risk | **Prediction:** Moderate.<br>**Recommended Test:** Since Patient B's peripheral artery disease risk is predicted to be moderate, blood flow status can be monitored through periodic **lower extremity Doppler ultrasound tests.** An additional test is not necessary, but lifestyle improvement to manage risk factors is recommended. |

When the processor of the diagnostic device obtains new data regarding the recommended tests, the processor of the diagnostic device may reevaluate the risk by cardiovascular disease type based thereon by re-inputting the corresponding patient's data into the second model (1200), as shown in FIG. 10.

### 3.2.4 Algorithm Reflecting Cardiovascular Plaque Types

The algorithm reflecting cardiovascular plaque types may be combined with the basic algorithm configuration introduced in section 3.2.1 above or with an algorithm that further includes the recommended test determination model of section 3.2.3. Since the content described above may be applied to the first model (cardiovascular risk prediction model, 1100) and the second model (risk prediction model by cardiovascular disease type, 1200), a description of the details will be omitted. As briefly mentioned above, the first model (1100) may learn the correlation between an inputted fundus image and cardiovascular plaque types. Cardiovascular plaque types may be obtained from CT coronary angiography (CT-CA), cardiovascular magnetic resonance (CMR), 18F-FDG PET, 18F-NaF PET, coronary angiography, intravascular ultrasound (IVUS), IVUS-RF analysis, optical coherence tomography (OCT), optical frequency domain imaging (OFDI), intracoronary thermography, Raman spectroscopy, or near infrared spectroscopy (NIRS). Specifically, the cardiovascular risk prediction model may be trained on labels indicating soft plaques and their corresponding retinal images, and labels indicating hard plaques and their corresponding retinal images. Accordingly, the cardiovascular risk prediction model may obtain a probability value (and/or grade) for the probability that the subject of the retinal image has soft plaques, or a probability value (and/or grade) for the probability that the subject of the retinal image has hard plaques. The first model can predict the plaque type as a probability value along with Reti-CVD and the disease type-specific factors, and the probability value can be expressed as a score.

The plaque type predicted from the first model (1100) can be reflected in predicting the risk by cardiovascular disease type. For example, in the case of soft plaques, the risk of acute events is high due to the possibility of rupture and embolism, and it may be particularly highly associated with stroke risk. In addition, soft plaques increase heart failure risk, and may increase the risk of peripheral artery disease as the risk of acute limb ischemia increases due to the possibility of embolization of the soft plaques. In contrast, in the case of hard plaques, they are associated with gradual occlusion and can lead to myocardial infarction disease over time. In addition, hard plaques increase heart failure risk, and may increase the risk of peripheral artery disease as the risk of chronic limb ischemia and intermittent claudication due to gradual narrowing of blood vessels increases.

Accordingly, the second model (1200) can predict risk by cardiovascular disease type, such as stroke, myocardial infarction, heart failure, or peripheral artery disease, by considering the plaque type predicted from the first model (1100) as a covariate by each detailed disease type. In addition, the third model (1300) can determine what clinical test is recommended for a patient by considering such plaque types. For example, if it is predicted that the risk of stroke disease is high from the second model and/or it is predicted that a soft plaque or a hard plaque exists from the third model, the third model can generate blood biomarkers (e.g., blood coagulation test, lipid profile), blood pressure information, carotid artery state information, or information necessary for diagnosis of atrial fibrillation disease (age, blood pressure information, etc.) as necessary information, and recommends clinical tests for acquiring the corresponding information. In addition, if it is confirmed that the risk of myocardial infarction disease is high and/or a hard plaque exists, the third model (1300) can generate blood biomarkers (e.g., lipid profile) or family history information as necessary information and recommends clinical tests for acquiring the corresponding information. In addition, if it is confirmed that the risk of heart failure disease is high based on heart failure disease diagnostic information and/or a soft plaque or a hard plaque exists, the third model (1300) can generate blood biomarkers (e.g., BNP level), history of previous myocardial infarction disease, or overall cardiac function indicators as necessary information, and recommends clinical tests for acquiring the corresponding information. In addition, if it is confirmed that the risk of peripheral artery disease is high based on peripheral artery disease diagnostic information and/or a soft plaque or a hard plaque exists, the third model can generate smoking information, diabetes information, or blood pressure information as necessary information, and recommends clinical tests for acquiring the corresponding information.

### 3.2.5 Algorithm for Predicting Risk by Sub-type of Each Cardiovascular Disease Type

Various forms (sub-types) exist for each cardiovascular disease type. Since each sub-type differs in the pathological mechanism, symptoms, prognosis, and treatment method of the disease, it is important to clearly distinguish them. The present invention goes beyond predicting the existing integrated cardiovascular risk through machine learning models based on fundus images, and provides algorithms for predicting risk by cardiovascular disease type. The processor of the diagnostic device can predict the risk by sub-type of each cardiovascular disease type based on the algorithms according to the present invention. Since the content described in FIG. 9 may be applied to the basic model configuration, a detailed description will be omitted.

As one example, in the case of heart failure (HF), sub-types of heart failure with reduced ejection fraction (HFrEF), heart failure with mid-range ejection fraction (HFmrEF), or heart failure with preserved ejection fraction (HFpEF) exist. Heart failure with reduced ejection fraction (HFrEF) is heart failure in a state where the left ventricular ejection fraction (LVEF) is reduced to 40% or less, where the heart's blood pumping capacity is greatly reduced due to a decrease in the contractile function of the myocardium. In this case, the disease type-specific factors correspond to previous myocardial infarction (MI), chronic hypertension, valvular disease, diabetes, and smoking, and an echocardiogram, a cardiac MRI, and a BNP (B-type natriuretic peptide) test may be recommended. Heart failure with preserved ejection fraction (HFpEF) is heart failure in a state where the left ventricular ejection fraction (LVEF) is preserved at 50% or more, mainly where there is a problem with the relaxation function of the ventricle, and the heart does not relax sufficiently, impairing the ability to fill blood. In this case, obesity, hypertension, diabetes, advanced age, and female gender can be considered as the disease type-specific factors, and an echocardiogram, a cardiac MRI, a BNP test, and the like may be recommended. In addition, heart failure with mid-range ejection fraction (HFmrEF) is heart failure in a state where the left ventricular ejection fraction (LVEF) is at a borderline of 40-49%, which is considered an intermediate form of HFrEF and HFpEF. Advanced age, hypertension, diabetes, obesity, and cardiac valvular disease can be considered as the disease type-specific factors, and an echocardiogram, a cardiac MRI, and a MBP test may be recommended.

Similar to the case of heart failure, stroke can be divided into ischemic stroke, thrombotic cerebral infarction (cerebral thrombosis), cerebral embolism (embolic cerebral infarction), lacunar infarction, hemorrhagic stroke, intracerebral hemorrhage, subarachnoid hemorrhage, transient ischemic attack, brainstem stroke, or stroke of unknown origin. Atrial fibrillation includes sub-types of paroxysmal atrial fibrillation, persistent atrial fibrillation, long-standing persistent atrial fibrillation, or permanent atrial fibrillation; and myocardial infarction is divided into sub-types of type 1 (spontaneous myocardial infarction), type 2 (myocardial infarction secondary to an ischemic imbalance), type 3 (myocardial infarction resulting in death when biomarker values are unavailable), type 4a (myocardial infarction related to percutaneous coronary intervention (PCI)), type 4b (myocardial infarction related to stent thrombosis), or type 5 (myocardial infarction related to coronary artery bypass grafting (CABG)).

Accordingly, the second model (risk prediction model by cardiovascular disease type) according to the present invention can predict the risk by sub-type according to disease type by reflecting the sub-type specific factors of each type in cardiovascular risk. In addition, through the third model (recommended test determination model), it is possible to recommend necessary clinical tests considering detailed sub-types for each type of cardiovascular disease for a target patient.

### 3.2.6 Secondary Auxiliary Information

Diagnostic information may include the aforementioned cardiovascular risk score (Reti-CVD), plaque-based diagnostic information, the target patient's disease type-specific factors, risk and related information for each detailed disease type, recommended test information, or the like. Here, secondary auxiliary information additionally generated based on the diagnostic information will be described. That is, the secondary auxiliary information includes secondary guide information obtained based on the diagnostic information. The secondary guide information may include, as one example, prescription information, action information, and management information.

Prescription information may refer to information on medications (e.g., prescription drugs) recommended to the subject to maintain or improve the cardiovascular risk according to the diagnostic information. Additionally, the prescription information may include information on a prescribed medication, timing of administration, and dosage. For example, the prescription information may include information on prescriptions for one or more of statin-class drugs (including various agents such as simvastatin, atorvastatin, or rosuvastatin), which are HMG-CoA reductase inhibitors, PCSK9 inhibitors, fibric acid derivative combination therapy, aspirin, bile acid sequestrants and nicotinic acid, omega-3 fatty acids, ezetimibe, or fibrates. Additionally, when information that soft plaques and/or hard plaques are present in the subject is included in the diagnostic information, the prescription information may include information on high-intensity statin therapy or calcium channel blockers or ACE inhibitors for hypertension management.

Additionally, action information may refer to information on future actions recommended for the subject to maintain or improve the cardiovascular risk according to the diagnostic information. For example, additional checkup information may include information on a secondary diagnosis or medical treatment for the subject. As one example, the additional checkup information may include information on an additionally required test, a hospital/medical staff where an additional test is available, or information on a recommended procedure/surgery.

Additionally, management information may include information on non-medical treatments recommended to the subject to maintain or improve the cardiovascular risk according to the diagnostic information. For example, the management information may include lifestyle habit information, dietary habit information, exercise information, or information on non-prescription products such as nutritional supplements for reducing cardiovascular disease risk. Additionally, when information that soft plaques are present in the subject is included in the diagnostic information, the management information may include information on strict lifestyle improvements (e.g., diet, exercise, smoking cessation, etc.).

Additionally, when information that hard plaques are present in the subject is included in the diagnostic information, the management information may include information on long-term lifestyle improvements. Additionally, in one embodiment, the diagnostic device may be interlocked with an external monitoring device. Here, the monitoring device may refer to a device for monitoring the subject's lifestyle habits or behaviors. For example, the monitoring device may include a portable device, a wearable device, a wellness measurement device, and the like. Additionally, the monitoring device may be the client device described above. For example, the monitoring device may include an imaging part, and can acquire images of the internal and external parts of the eye by photographing the internal and external parts of the eye through the imaging part to obtain a fundus image.

And, the monitoring device can monitor various information, such as the subject's activity level, exercise method, exercise duration, food consumed, intake amount, health supplement intake information, sleep duration, sleep habits, heart rate, blood pressure, blood glucose levels, body water content, oxygen levels, body temperature, oxygen saturation, pulse wave, whether or not visiting a hospital, whether or not a test was received, whether or not a procedure/surgery was received, or ocular images.

The processor of the diagnostic device can perform communication with the monitoring device wiredly or wirelessly through the communication module (13).

The processor of the diagnostic device can provide guide information to the monitoring device. And, the monitoring device can provide various information to the subject based on the guide information and monitored information. For example, the monitoring device may obtain management information (e.g., lifestyle habit information, dietary habit information, exercise information) as guide information from the diagnostic device, compare the management information with the monitored information, determine whether the monitored information matches the management information, and provide a judgment result and/or additional information accordingly.

For example, if a monitored exercise time is less than an exercise time of the management information, the monitoring device can provide information to the subject to exercise according to the management information. Additionally, if food intake among the monitored information corresponds to food intake of the management information, the monitoring device can provide information to the subject that food intake is being well performed according to the management information.

Additionally, the processor of the diagnostic device can obtain monitored information from the monitoring device. The processor of the diagnostic device can provide various information to the subject based on the guide information and the monitored information. For example, the processor of the diagnostic device compares monitored information with guide information to determine whether the monitored information matches the management information, and can provide a judgment result and/or additional information accordingly. The aforementioned example of the monitoring device can be applied to the operation of the processor of the diagnostic device.

Additionally, the processor of the diagnostic device can generate guide information by reflecting monitoring information received from the monitoring device. For example, the processor of the diagnostic device acquires state information of the subject (exercise state, lifestyle state, eating habit state, etc.) based on the monitoring information, and can modify guide information determined as diagnostic information to suit the subject based on the state information of the subject.

In one embodiment, the processor of the diagnostic device can provide guide information using a predetermined database. For example, the diagnostic device may include a database matching scores and/or grades of diagnostic information with guide information. For example, when diagnostic information is expressed in three grades, the database may include: guide information matched with a low risk group (e.g., prescription information-none; action information-information on the next medical treatment period; management information-providing dietary habit information, providing exercise information); guide information matched with a moderate risk group (e.g., prescription information-none; action information-providing additional test information; management information-providing dietary habit information, providing exercise information, providing non-prescription product information); and guide information matched with a high risk group (e.g., prescription information-providing statin prescription information; action information-additional test information, providing recommended procedure/surgery information; management information-providing dietary habit information, providing exercise information, providing non-prescription product information). The processor of the diagnostic device can provide guide information matching the diagnostic information based on the database.

Additionally, in another embodiment, the processor of the diagnostic device can provide guide information using a machine learning model. For example, the diagnostic device may include a guide information model based on a machine learning model or a neural network model. The guide information model can be trained on scores and/or grades of diagnostic information and guide information. Additionally, the guide information model can also be trained on at least one of the subject's physical information (at least one of height, weight, age, gender, race, smoking status, blood pressure (e.g., blood pressure value, whether or not hypertension), whether or not diabetes (or blood glucose value), or cholesterol value). Accordingly, the processor of the diagnostic device can obtain guide information for the subject by inputting scores and/or grades of diagnostic information and the subject's physical information into the guide information model. Additionally, according to an embodiment, the guide information model may be included in the aforementioned diagnostic model or may be configured independently of the diagnostic model.

Various embodiments of the present specification may be implemented as software including instructions stored in a machine-readable storage medium (e.g., a computer). A machine is a device capable of calling instructions stored from a storage medium and operating according to the called instructions, and may include an electronic device according to the disclosed embodiments. When the command is executed by a processor, the processor can perform a function corresponding to the command directly or by using other components under the control of the processor. The instructions may include code generated or executed by a compiler or an interpreter. A machine-readable storage medium may be provided in the form of a non-transitory storage medium. Here, 'non-transitory storage medium' merely means that it does not include a signal and is tangible, and does not distinguish that data is semi-permanently or temporarily stored in the storage medium. For example, 'non-transitory storage medium' may include a buffer in which data is temporarily stored.

According to one embodiment, a method according to various embodiments disclosed in the present specification may be provided by being included in a computer program product. The computer program product can be traded between a seller and a buyer as a product. The computer program product may be distributed in the form of a machine-readable storage medium (e.g., Compact Disc Read Only Memory, CD-ROM) or through an application store (e.g., Play Store™). In the case of online distribution, at least a part of a computer program product, for example, a downloadable app, may be at least temporarily stored or temporarily generated in a storage medium such as a memory of a manufacturer's server, an application store's server, or a relay server.

As described above, although embodiments have been described with reference to limited embodiments and drawings, various modifications and variations are possible from the above description by those of ordinary skill in the art. For example, appropriate results can be achieved even if the described techniques are performed in a different order from a described method, and/or components such as systems, structures, devices, circuits, etc. described are combined or assembled in a different form from a described method, or are replaced or substituted by other components or equivalents.

Therefore, other implementations, other embodiments, and things equivalent to the patent claims also fall within the scope of the following patent claims.

**Claims**

1. A device for predicting and diagnosing risk by cardiovascular disease type, comprising:

   at least one processor, wherein the at least one processor predicts risk by cardiovascular disease type using a diagnostic model,
   wherein the diagnostic model comprises:

   a first model that analyzes an inputted fundus image to predict cardiovascular risk; and
   a second model that receives as inputs a cardiovascular risk score predicted by the first model and disease type-specific factors, and predicts risk by cardiovascular disease type,
   wherein the at least one processor is **characterized by** providing diagnostic information based on the cardiovascular risk predicted by the first model and the risk by cardiovascular disease type predicted by the second model.

2. The device according to claim 1, **characterized in that** sub-types of the cardiovascular disease include at least one of myocardial infarction (MI), peripheral artery disease (PAD), stroke, atrial fibrillation (AF), or heart failure (HF).

3. The device according to claim 1, **characterized in that** the disease type-specific factors include at least one of a patient's age, gender, blood biomarkers, urinalysis values, smoking status, whether or not obese, electrocardiogram (ECG), or patient's family history.

4. The device according to claim 1, **characterized in that** the disease type-specific factors are predicted by the first model or are input from an external source.

5. The device according to claim 1, **characterized in that** the diagnostic information includes a risk grade for each cardiovascular type and related information.

6. A device for predicting and diagnosing risk by cardiovascular disease type, comprising:

   at least one processor,
   wherein the at least one processor provides cardiovascular diagnostic information using a diagnostic model,
   wherein the diagnostic model comprises:

   a first model that analyzes an inputted fundus image to predict cardiovascular risk;
   a second model that receives as inputs a cardiovascular risk score predicted by the first diagnostic model and disease type-specific factors, and predicts risk by cardiovascular disease type; and
   a third model that determines a clinical test recommended for a patient based on the risk by cardiovascular disease type predicted by the second model,
   wherein the at least one processor is **characterized by** providing the cardiovascular diagnostic information based on the cardiovascular risk predicted by the first model and the risk by cardiovascular disease type predicted by the second model.

7. The device according to claim 6, **characterized in that** sub-types of the cardiovascular disease include at least one of myocardial infarction (MI), peripheral artery disease (PAD), stroke, atrial fibrillation (AF), or heart failure (HF).

8. The device according to claim 6, **characterized in that** the disease type-specific factors include at least one of a patient's age, gender, blood biomarkers, smoking status, whether or not obese, urinalysis values, electrocardiogram (ECG), or patient's family history.

9. The device according to claim 6, **characterized in that** the disease type-specific factors are predicted by the first model or are input from an external source.

10. The device according to claim 6, **characterized in that** additional clinical tests include at least one of an electrocardiogram (ECG), an echocardiogram, an ankle-brachial index (ABI), an ankle-brachial pressure index (ABPI), a blood test, a urinalysis, or an ultrasound.

11. The device according to claim 6, **characterized in that** the cardiovascular diagnostic information includes a risk grade and related information for each disease type, or information related to recommended tests.

12. A device for predicting and diagnosing risk by cardiovascular disease type, comprising:

at least one processor,
wherein the at least one processor provides cardiovascular diagnostic information using a diagnostic model,
wherein the diagnostic model comprises:

a first model that analyzes an inputted fundus image to predict cardiovascular risk and a cardiovascular plaque type;
a second model that receives as inputs a cardiovascular risk score predicted by the first model and disease type-specific factors, and predicts risk by cardiovascular disease type; and
a third model that determines a clinical test recommended for a patient based on the risk by cardiovascular disease type predicted by the second model,
**characterized by** providing the cardiovascular diagnostic information based on results predicted from the first to third models.

13. The device according to claim 12, **characterized in that** sub-types of the cardiovascular disease include at least one of myocardial infarction (MI), peripheral artery disease (PAD), stroke, atrial fibrillation (AF), or heart failure (HF).

14. The device according to claim 12, **characterized in that** the disease type-specific factors include at least one of a patient's age, gender, blood biomarkers, smoking status, whether or not obese, urinalysis values, electrocardiogram (ECG), or patient's family history.

15. The device according to claim 12, **characterized in that** the disease type-specific factors are predicted by the first model or are input from an external source.

16. The device according to claim 12, **characterized in that** additional clinical tests include at least one of an electrocardiogram (ECG), an echocardiogram, an ankle-brachial index (ABI), an ankle-brachial pressure index (ABPI), a blood test, a urinalysis, or an ultrasound.

17. The device according to claim 12, **characterized in that** the cardiovascular diagnostic information includes a risk grade and related information for each cardiovascular disease type, a plaque type, or information related to recommended tests.

18. The device according to claim 15, **characterized in that**, in a case where the disease type-specific factors are input into the second model from an external source, the risk for each cardiovascular disease type is re-evaluated based on the disease type-specific factors obtained from the external source.

19. A device for predicting and diagnosing risk by cardiovascular disease type, comprising:

at least one processor,
wherein the at least one processor predicts a risk for each cardiovascular disease type using a diagnostic model,
wherein the diagnostic model comprises:

a first model that analyzes an inputted fundus image to predict cardiovascular risk; and
a second model that receives as inputs a cardiovascular risk score predicted by the first model and sub-type specific factors for each disease type, and predicts a risk for each sub-type of the disease type,
wherein the at least one processor is **characterized by** providing diagnostic information based on the cardiovascular risk predicted by the first model and the risk for each sub-type of the type predicted by the second model.

20. The device according to claim 19, **characterized in that** the cardiovascular disease types include at least one of myocardial infarction (MI), peripheral artery disease (PAD), stroke, atrial fibrillation (AF), or heart failure (HF).

21. The device according to claim 20, **characterized in that**:

the heart failure includes one or more sub-types among heart failure with reduced ejection fraction (HFrEF), heart failure with mid-range ejection fraction (HFmrEF), or heart failure with preserved ejection fraction (HFpEF);
the stroke includes one or more sub-types among ischemic stroke, thrombotic cerebral infarction (cerebral thrombosis), cerebral embolism (embolic cerebral infarction), lacunar infarction, hemorrhagic stroke, intracerebral hemorrhage, subarachnoid hemorrhage, transient ischemic attack, brainstem stroke, or stroke of unknown origin;
the atrial fibrillation includes one or more sub-types among paroxysmal atrial fibrillation, persistent atrial fibrillation, long-standing persistent atrial fibrillation, or permanent atrial fibrillation; and
the myocardial infarction includes one or more sub-types among type 1 (spontaneous myocardial infarction), type 2 (myocardial infarction secondary to an ischemic imbalance), type 3 (myocardial infarction resulting in death when biomarker values are unavailable), type 4a (myocardial infarction related to percutaneous coronary intervention (PCI)), type 4b (myocardial infarction related to stent thrombosis), or type 5 (myocardial infarction related to coronary artery bypass grafting (CABG)).

<u>10</u>

11              12              13

| STORAGE MODULE | ◄──► | PROCESSOR | ◄──► | COMMUNICATION MODULE |
|---|---|---|---|---|

# FIG.1

| Reti-CVD | | STROKE | | | MYOCARDIAL INFARCTION | | | ATRIAL FIBRILLATION | | |
|---|---|---|---|---|---|---|---|---|---|---|
| RISK GROUP | POPULATION | EVENT | (%) | INCIDENCE | EVENT | (%) | INCIDENCE | EVENT | (%) | INCIDENCE |
| LOW RISK GROUP | 17606 | 28 | (0.16) | 0.23 | 79 | (0.45) | 0.64 | 135 | (0.77) | 1.10 |
| MODERATE RISK GROUP | 24180 | 198 | (0.82) | 1.19 | 343 | (1.42) | 2.06 | 661 | (2.73) | 3.99 |
| HIGH RISK GROUP | 2279 | 30 | (1.32) | 1.93 | 64 | (2.81) | 4.14 | 153 | (6.71) | 10.06 |
| TOTAL | 44065 | 256 | (0.58) | 0.84 | 486 | (1.10) | 1.60 | 949 | (2.15) | 3.13 |

# FIG.2A

| | STROKE | | | MYOCARDIAL INFARCTION | | | ATRIAL FIBRILLATION | | |
|---|---|---|---|---|---|---|---|---|---|
| Reti-CVD | ADJUSTED HR (95% CI) | | | ADJUSTED HR (95% CI) | | | ADJUSTED HR (95% CI) | | |
| LOW RISK GROUP | 1 (REFERENCE) | | | 1 (REFERENCE) | | | 1 (REFERENCE) | | |
| MODERATE RISK GROUP | 2.34( | 1.51 - | 3.63) | 1.20( | 0.91 - | 1.58) | 1.19( | 0.97 - | 1.47) |
| HIGH RISK GROUP | 2.45( | 1.37 - | 4.39) | 1.49( | 1.03 - | 2.15) | 1.70( | 1.30 - | 2.23) |
| HR TREND | 1.50( | 1.16 - | 1.95) | 1.22( | 1.01 - | 1.47) | 1.32( | 1.15 - | 1.51) |
| C-INDEX | 0.75( | 0.72 - | 0.78) | 0.78( | 0.76 - | 0.80) | 0.77( | 0.75 - | 0.78) |

# FIG.2B

| DISEASE | Reti-CVD | OR | LCL    UCL | P |
|---|---|---|---|---|
| CORONARY ARTERY | LOW RISK GROUP | 1 (REFERENCE) | | |
| | MODERATE RISK GROUP | 2.22 | (1.58 - 3.12) | <0.001 |
| | HIGH RISK GROUP | 10.37 | (7.58 - 14.18) | <0.001 |
| | TREND | 3.91 | (3.44 - 4.44) | <0.001 |
| PERIPHERAL VASCULAR DISEASE | LOW RISK GROUP | 1 (REFERENCE) | | |
| | MODERATE RISK GROUP | 2.33 | (0.64 - 8.44) | 1.98 |
| | HIGH RISK GROUP | 9.65 | (2.94 - 31.64) | <0.001 |
| | TREND | 3.59 | (2.22 - 5.80) | <0.001 |
| ATRIAL FIBRILLATION | LOW RISK GROUP | 1 (REFERENCE) | | |
| | MODERATE RISK GROUP | 2.16 | (1.45 - 3.23) | <0.001 |
| | HIGH RISK GROUP | 9.36 | (6.51 - 13.45) | <0.001 |
| | TREND | 3.52 | (3.00 - 4.14) | <0.001 |
| AORTIC VALVE STENOSIS | LOW RISK GROUP | 1 (REFERENCE) | | |
| | MODERATE RISK GROUP | 2.32 | (0.48 - 11.35) | 0.298 |
| | HIGH RISK GROUP | 8.13 | (1.87 - 35.35) | 0.005 |
| | TREND | 3.14 | (1.72 - 5.74) | <0.001 |

# FIG.3

| DISEASE | Reti-CVD | OR | LCL UCL | P |
|---|---|---|---|---|
| HEART FAILURE | LOW RISK GROUP | 1 (REFERENCE) | | |
| | MODERATE RISK GROUP | 1.68 | (0.77 - 3.68) | 0.194 |
| | HIGH RISK GROUP | 7.33 | (3.64 - 14.77) | <0.001 |
| | TREND | 3.31 | (2.42 - 4.54) | <0.001 |
| ISCHEMIC STROKE | LOW RISK GROUP | 1 (REFERENCE) | | |
| | MODERATE RISK GROUP | 1.99 | (0.93 - 4.25) | 0.077 |
| | HIGH RISK GROUP | 4.7 | (2.30 - 9.59) | <0.001 |
| | TREND | 2.24 | (1.66 - 3.04) | <0.001 |
| TRANSIENT ISCHEMIC ATTACK | LOW RISK GROUP | 1 (REFERENCE) | | |
| | MODERATE RISK GROUP | 1.33 | (0.61 - 2.92) | 0.478 |
| | HIGH RISK GROUP | 4.17 | (2.07 - 8.40) | <0.001 |
| | TREND | 2.34 | (1.67 - 3.27) | <0.001 |
| ARTERIAL HYPERTENSION | LOW RISK GROUP | 1 (REFERENCE) | | |
| | MODERATE RISK GROUP | 1.67 | (1.45 - 1.92) | <0.001 |
| | HIGH RISK GROUP | 3.17 | (2.78 - 3.61) | <0.001 |
| | TREND | 1.82 | (1.72 - 1.93) | <0.001 |
| PULMONARY EMBOLISM | LOW RISK GROUP | 1 (REFERENCE) | | |
| | MODERATE RISK GROUP | 1.45 | (0.87 - 2.41) | 0.156 |
| | HIGH RISK GROUP | 3.00 | (1.86 - 4.84) | <0.001 |
| | TREND | 1.79 | (1.42 - 2.27) | <0.001 |
| DEEP VEIN THROMBOSIS | LOW RISK GROUP | 1 (REFERENCE) | | |
| | MODERATE RISK GROUP | 1.60 | (1.06 - 2.42) | 0.025 |
| | HIGH RISK GROUP | 2.54 | (1.70 - 3.80) | <0.001 |
| | TREND | 1.59 | (1.31 - 1.93) | <0.001 |
| CEREBROVASCULAR DISEASE | LOW RISK GROUP | 1 (REFERENCE) | | |
| | MODERATE RISK GROUP | 1.06 | (0.70 - 1.60) | 0.787 |
| | HIGH RISK GROUP | 2.36 | (1.62 - 3.42) | <0.001 |
| | TREND | 1.68 | (1.40 - 2.02) | <0.001 |

# FIG.4

|  | Reti-CVD | | |
|---|---|---|---|
| VARIABLE | LOW RISK GROUP (n=550) | MODERATE RISK GROUP (n=276) | HIGH RISK GROUP (n=275) |
| Reti-CVD | 45 (8.2%) | 42 (15.2%) | 51 (18.5%) |
| AGE, MEAN (STANDARD DEVIATION), YEARS | 55.6 (7.9) | 61.2 (6.1) | 63.3 (5.2) |
| SEX, NUMBER (%) | 231 (42.0%) | 177 (64.1%) | 193 (70.2%) |
| ANTIHYPERTENSIVE MEDICATION, NUMBER (%) | 192 (34.9) | 151 (54.7%) | 149 (54.2%) |
| STATIN, NUMBER (%) | 114 (20.7%) | 52 (18.8%) | 44 (16.0%) |
| LIFETIME SMOKER, NUMBER (%) | 187 (34.0%) | 121 (43.8%) | 153 (55.6%) |

## FIG.5A

| Reti-CVD | POPULATION | EVENT | INCIDENCE (95% CONFIDENCE INTERVAL) | aHR(95% CI)* | P-VALUE |
|---|---|---|---|---|---|
| LOW RISK GROUP | 550 | 45 | 7.7 (5.8-10.4) | | |
| MODERATE RISK GROUP | 276 | 42 | 15.2 (11.2-20.6) | 1.57 (1.00-2.47) | 0.050 |
| HIGH RISK GROUP | 275 | 51 | 19.0 (14.4-25.0) | 1.88 (1.19-2.98) | 0.007 |
| | 1,101 | 138 | 12.3 (10.4-14.5) | | |
| | | | | HR TREND = 1.36 (1.09-1.70) | 0.007 |

## FIG.5B

FIG.6

START

ACQUIRING FUNDUS IMAGE ~S100

OBTAINING CARDIOVASCULAR
DISEASE DIAGNOSTIC INFORMATION ~S200

END

# FIG.7

| | HR | LCL | | UCL | | P |
|---|---|---|---|---|---|---|
| Reti-AF | | | | | | |
| LOW RISK GROUP | | REFERENCE | | | | |
| MODERATE RISK GROUP | | ( 1.95 | - | 3.71 | ) | <0.01 |
| HIGH RISK GROUP | | ( 4.08 | - | 7.41 | ) | <0.01 |
| (TREND) | | ( 1.98 | - | 2.57 | ) | <0.01 |
| C-INDEX | | ( 0.64 | - | 0.68 | ) | |

# FIG.8A

| | HR | LCL | | UCL | | P |
|---|---|---|---|---|---|---|
| Reti-AF | | | | | | |
| LOW RISK GROUP | | REFERENCE | | | | |
| MODERATE RISK GROUP | 1.14 | ( 0.80 | - | 1.61 | ) | 0.47 |
| HIGH RISK GROUP | 1.24 | ( 0.86 | - | 1.78 | ) | 0.26 |
| (TREND) | 1.10 | ( 0.94 | - | 1.30 | ) | 0.24 |
| AGE | 1.11 | ( 1.09 | - | 1.13 | ) | <0.01 |
| SEX | | | | | | |
| FEMALE | | REFERENCE | | | | |
| MALE | 1.69 | ( 1.47 | - | 1.94 | ) | <0.01 |
| SMOKING | 0.67 | ( 0.44 | - | 1.02 | ) | 0.06 |
| DIABETES | 1.53 | ( 1.06 | - | 2.19 | ) | 0.02 |
| HYPERLIPIDEMIA | 0.85 | ( 0.71 | - | 1.03 | ) | 0.10 |
| HYPERTENSION | 1.29 | ( 1.05 | - | 1.59 | ) | 0.02 |
| C-INDEX | 0.76 | ( 0.74 | - | 0.78 | ) | |

# FIG.8B

1000

DIAGNOSTIC MODEL

1100                    1200

INPUT → FIRST MODEL → SECOND MODEL → CARDIOVASCULAR DISEASE DIAGNOSTIC INFORMATION

# FIG.9

1000

DIAGNOSTIC MODEL

1100                    1200

INPUT → FIRST MODEL → SECOND MODEL → CARDIOVASCULAR DISEASE DIAGNOSTIC INFORMATION

1300 ~ THIRD MODEL

# FIG.10

## INTERNATIONAL SEARCH REPORT

| | International application No. |
|---|---|
| | **PCT/KR2024/012729** |

| A. | CLASSIFICATION OF SUBJECT MATTER |
|---|---|

**A61B 3/00**(2006.01)i; **A61B 3/12**(2006.01)i; **A61B 3/14**(2006.01)i; **G16H 50/20**(2018.01)i; **G16H 50/50**(2018.01)i; **G16H 30/20**(2018.01)i

According to International Patent Classification (IPC) or to both national classification and IPC

| B. | FIELDS SEARCHED |
|---|---|

Minimum documentation searched (classification system followed by classification symbols)

A61B 3/00(2006.01); A61B 5/00(2006.01); A61B 5/318(2021.01); G06N 7/00(2006.01); G06Q 40/08(2012.01); G16H 10/20(2018.01); G16H 20/70(2018.01); G16H 50/20(2018.01); G16H 50/30(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 안저 사진(fundus image), 심혈관(cardiovascular), 위험도(risk), 분석 (analyze), 예측(predict), 모델(model), 진단(diagnosis)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-2020-0005411 A (UIF (UNIVERSITY INDUSTRY FOUNDATION), YONSEI UNIVERSITY et al.) 15 January 2020 (2020-01-15)<br>See paragraphs [0527]-[0859]. | 1,3-5,19 |
| Y | | 2,6-18,20,21 |
| Y | KR 10-2555479 B1 (MEDIBLOC CO., LTD. et al.) 17 July 2023 (2023-07-17)<br>See paragraph [0029]. | 2,7,9,13,15,18,20,21 |
| Y | KR 10-2022-0080300 A (LEE, Mi Kyoung) 14 June 2022 (2022-06-14)<br>See claim 5. | 6-18 |
| Y | KR 10-2022-0079076 A (AI PLATFORM CO., LTD.) 13 June 2022 (2022-06-13)<br>See paragraph [0023]. | 12-18 |

| ✓ Further documents are listed in the continuation of Box C. | ✓ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 December 2024** | **02 December 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2024/012729** |

**C. DOCUMENTS CONSIDERED TO BE RELEVANT**

| Category* | Citation of document, with indication, where appropriate, of the relevant passages | Relevant to claim No. |
| --- | --- | --- |
| A | KR 10-2021-0053545 A (SELVAS AI INC.) 12 May 2021 (2021-05-12)<br>See paragraph [0070]. | 1-21 |

Form PCT/ISA/210 (second sheet) (July 2022)

**INTERNATIONAL SEARCH REPORT**
Information on patent family members

International application No.

**PCT/KR2024/012729**

| Patent document cited in search report | | | Publication date (day/month/year) | Patent family member(s) | | | Publication date (day/month/year) |
|---|---|---|---|---|---|---|---|
| KR | 10-2020-0005411 | A | 15 January 2020 | KR | 10-2020-0005404 | A | 15 January 2020 |
| | | | | KR | 10-2020-0005405 | A | 15 January 2020 |
| | | | | KR | 10-2020-0005406 | A | 15 January 2020 |
| | | | | KR | 10-2020-0005407 | A | 15 January 2020 |
| | | | | KR | 10-2020-0005408 | A | 15 January 2020 |
| | | | | KR | 10-2020-0005409 | A | 15 January 2020 |
| | | | | KR | 10-2020-0005412 | A | 15 January 2020 |
| | | | | KR | 10-2020-0005413 | A | 15 January 2020 |
| | | | | KR | 10-2020-0005433 | A | 15 January 2020 |
| | | | | KR | 10-2021-0158853 | A | 31 December 2021 |
| | | | | KR | 10-2023-0110233 | A | 21 July 2023 |
| | | | | KR | 10-2212500 | B1 | 04 February 2021 |
| | | | | KR | 10-2270659 | B1 | 29 June 2021 |
| | | | | KR | 10-2345026 | B1 | 29 December 2021 |
| KR | 10-2555479 | B1 | 17 July 2023 | KR | 10-2021-0151584 | A | 14 December 2021 |
| KR | 10-2022-0080300 | A | 14 June 2022 | KR | 10-2698450 | B1 | 22 August 2024 |
| KR | 10-2022-0079076 | A | 13 June 2022 | KR | 10-2418399 | B1 | 07 July 2022 |
| KR | 10-2021-0053545 | A | 12 May 2021 | | None | | |

Form PCT/ISA/210 (patent family annex) (July 2022)

# EP 4 767 922 A1

**Patent documents cited in the description**

- KR 1020180166720
- KR 1020180166721
- KR 1020180166722